# EUROPÄISCHE PATENTANMELDUNG

(11) **EP 4 043 877 A1**
(43) Veröffentlichungstag der Anmeldung: **17.08.2022**
(21) Anmeldenummer: 21156881.1
(22) Anmeldetag: 12.02.2021
(51) Int. Cl.: G01N 33/00, B60H 1/00, F24F 110/70

(54) **SENSORSYSTEM UND VERFAHREN ZUM UEBERWACHEN EINER UMWELTGROESSE**

(71) Anmelder: eesy-innovation GmbH, 82008 Unterhaching (DE)
(72) Erfinder: CORONA, Angel, 81541 München (DE)
(74) Vertreter: Bobbert & Partner Patentanwälte PartmbB

(57) **Zusammenfassung**

Die Erfindung betrifft ein Sensorsystem zum Überwachen wenigstens einer Umweltgröße, wobei das Sensorsystem wenigstens eine Erfassungseinrichtung, umfassend wenigstens einen Sensor, der wenigstens zum Ermitteln wenigstens einer Umweltgröße, insbesondere wenigstens einer CO2-Konzentration, ausgebildet ist und wobei die Erfassungseinrichtung wenigstens eine Kommunikationsschnittstelle aufweist und/oder wobei das Sensorsystem ausgebildet und/oder konfiguriert ist, ein Ausgangssignal zu erzeugen, welches wenigstens einen ermittelten Wert der wenigstens einen Umweltgröße berücksichtigt. Sie betrifft ferner ein entsprechendes Verfahren, eine Steuer- und/oder Regeleinrichtung, ein digitales Speichermedium, ein Computerprogramm-Produkt sowie ein Computerprogramm.

## Beschreibung

Die Erfindung betrifft ein Sensorsystem zum Überwachen wenigstens einer Umweltgröße gemäß dem Oberbegriff des Anspruchs 1 sowie ein Verfahren gemäß dem Oberbegriff des Anspruchs 10. Zudem betrifft sie eine Steuer- und/oder Regeleinrichtung gemäß Anspruch 11 sowie ein digitales Speichermedium gemäß Anspruch 12, ein Computerprogramm-Produkt gemäß Anspruch 13 sowie ein Computerprogramm gemäß Anspruch 14.

Beim Ausatmen werden winzige Tröpfchen, Aerosole, in der Luftumgebung freigesetzt. Aerosole können sich unter Umständen bis zu mehreren Stunden in der Luft halten. In geschlossenen oder im wesentlich geschlossenen Räumen, in welchen sich beispielsweise Menschen aufhalten, erhöht sich deren Konzentration in der Luft mit der Zeit. Beim Ausatmen wird zudem CO2 in der Luftumgebung freigesetzt, deren Konzentration mit der Zeit, unter anderem abhängig von der Personenzahl und der Raumgröße, ebenfalls zunimmt. Besagte Aerosole können mit Viren, beispielsweise Sars CoV-2, belastet sein. Die Überwachung der CO2-Konzentration, insbesondere in einem Raum, ermöglicht somit eine Abschätzung einer potenziellen Virenbelastung.

Zudem kann eine erhöhte CO2-Konzentration an sich allein schon Kopfschmerzen, Müdigkeit, Unruhe, vermehrtes Schwitzen, eine erhöhte Herzfrequenz, erhöhte Blutdruckwerte und/oder eine verminderte Konzentrationsfähigkeit verursachen. CO2-Konzentrationen ab 20.000 ppm (parts per million, 10⁻⁶) können weiterhin Husten verursachen. CO2-Konzentrationen um 100.000 ppm können Bewusstlosigkeit verursachen. Schließlich herrscht ab CO2-Konzentrationen über 200.000 ppm Erstickungsgefahr, da der Sauerstoff durch CO2 aus der Blutbahn verdrängt wird.

Die Überwachung der CO2-Konzentration kann somit als Indikator für die Luftqualität verwendet werden.

Eine erfindungsgemäße Aufgabe kann darin bestehen, ein weiteres Verfahren und/oder Sensorsystem zum Ermitteln und/oder Überwachen einer Umweltgröße, insbesondere einer CO2-Konzentration, vorzuschlagen. Die Aufgabe kann außerdem daraus bestehen, eine weitere Steuer- und/oder Regeleinrichtung, ein weiteres digitales Speichermedium, ein weiteres Computerprogramm-Produkt sowie ein weiteres Computerprogramm vorzuschlagen.

Die Aufgabe kann gelöst werden durch ein Sensorsystem zum Überwachen wenigstens einer Umweltgröße gemäß dem Oberbegriff des Anspruchs 1 sowie durch ein Verfahren gemäß dem Oberbegriff des Anspruchs 10. Ferner kann die Aufgabe mittels einer Steuer- und/oder Regeleinrichtung mit den Merkmalen des Anspruchs 11 sowie mittels eines digitalen Speichermediums mit den Merkmalen des Anspruchs 12, eines Computerprogramm-Produkts mit den Merkmalen des Anspruchs 13 und mittels eines Computerprogramms mit den Merkmalen des Anspruchs 14 gelöst werden.

Das Sensorsystem der vorliegenden Erfindung weist wenigstens eine Erfassungseinrichtung mit wenigstens einem Sensor und/oder mit wenigstens einem Sensormodul, auf. Der Sensor und/oder das Sensormodul ist wenigstens zum Ermitteln wenigstens einer Umweltgröße ausgebildet, programmiert und/oder konfiguriert.

Weiterhin kann das Sensorsystem und/oder die Erfassungseinrichtung wenigstens eine Kommunikationsschnittstelle aufweisen.

Alternativ oder ergänzend ist das Sensorsystem ausgebildet und/oder konfiguriert, ein Ausgangssignal zu erzeugen, welches wenigstens einen ermittelten Wert der wenigstens einen Umweltgröße berücksichtigt und/oder auf diesen basiert.

Das erfindungsgemäße Verfahren ist geeignet und/oder vorgesehen zum Überwachen und/oder Ermitteln wenigstens einer Umweltgröße, und/oder zum Erzeugen und/oder zum Ausgeben eines Ausgangssignals, welches wenigstens einen ermittelten Wert wenigstens einer Umweltgröße berücksichtigt und/oder auf diesem basiert.

Die erfindungsgemäße Steuer- und/oder Regeleinrichtung ist geeignet und vorgesehen und/oder ausgelegt und/oder konfiguriert zur Durchführung des erfindungsgemäßen Verfahrens, insbesondere im Zusammenwirken mit den hierzu jeweils erforderlichen Einrichtungen, beispielsweise dem erfindungsgemäßen Sensorsystem und/oder Teilen hiervon, vorzugsweise wie im Folgenden beschrieben.

Ein erfindungsgemäßes digitales, insbesondere nichtflüchtiges, Speichermedium, insbesondere in Form eines maschinenlesbaren Trägers, insbesondere in Form einer Diskette, CD, EPROM oder DVD, mit elektrisch auslesbaren Steuersignalen kann derart mit einem programmierbaren Computersystem zusammenwirken, dass die maschinellen Schritte eines erfindungsgemäßen Verfahrens veranlasst werden.

Dabei können alle, einige oder manche der maschinell durchgeführten Schritte des erfindungsgemäßen Verfahrens veranlasst werden.

Ein erfindungsgemäßes Computerprogramm-Produkt weist einen volatilen, flüchtigen oder auf einem maschinenlesbaren Träger gespeicherten Programmcode zur Veranlassung der maschinellen Schritte des erfindungsgemäßen Verfahrens, wenn das Computerprogramm-Produkt auf einem Rechner abläuft, auf. Unter einem Computerprogramm-Produkt kann erfindungsgemäß beispielsweise ein auf einem Träger gespeichertes Computerprogramm, ein Embedded System als umfassendes System mit einem Computerprogramm (z. B. elektronisches Gerät mit einem Computerprogramm), ein Netzwerk von computerimplementierten Computerprogrammen (z. B. Client/Serversystem, Cloud Computing System, etc.), oder ein Computer, auf dem ein Computerprogramm geladen ist, abläuft, gespeichert ist, ausgeführt oder entwickelt wird, verstanden werden.

Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte oder dergleichen sein.

Ein erfindungsgemäßes Computerprogramm weist einen Programmcode auf zur Veranlassung der maschinellen Schritte eines erfindungsgemäßen Verfahrens, wenn das Computerprogramm auf einem Computer abläuft. Unter einem Computerprogramm kann erfindungsgemäß beispielsweise ein physikalisches, vertriebsfähiges Software-Produkt verstanden werden, welches ein Programm aufweist.

Auch für das erfindungsgemäße Computerprogramm-Produkt und das erfindungsgemäße Computerprogramm gilt, dass alle, einige oder manche der maschinell durchgeführten Schritte des erfindungsgemäßen Verfahrens veranlasst werden.

Erfindungsgemäße Ausführungsformen können manche, einige oder alle der folgenden Merkmale in beliebiger Kombination aufweisen, soweit dies für den Fachmann nicht erkennbar technisch unmöglich ist. Vorteilhafte Weiterbildungen der vorliegenden Erfindung sind jeweils auch Gegenstand der Unteransprüche.

Bei allen hierin gemachten Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Hierin gemachte räumliche Angaben wie "oben", "unten", usw. beziehen sich im Zweifel auf die Darstellung, wie sie den hier beigefügten Figuren zu entnehmen ist.

Wenn hierin von einer Ausführungsform die Rede ist, so stellt diese stets eine erfindungsgemäße, beispielhafte Ausführungsform dar, die nicht als beschränkend zu verstehen ist.

Wenn hierin offenbart ist, dass der erfindungsgemäße Gegenstand ein oder mehrere Merkmale in einer bestimmten Ausführungsform aufweist, so ist hierin jeweils auch offenbart, dass der erfindungsgemäße Gegenstand genau dieses oder diese Merkmale in anderen, ebenfalls erfindungsgemäßen Ausführungsformen ausdrücklich nicht aufweist, z. B. im Sinne eines Disclaimers. Für jede hierin genannte Ausführungsform gilt somit, dass die gegenteilige Ausführungsform, beispielsweise als Negation formuliert, ebenfalls offenbart ist.

Bevorzugte Anwendungsgebiete und/oder -räume der vorliegenden Erfindung können beispielsweise Büros, insbesondere Großraumbüros, Klassenzimmer, Kindergärten, Geschäfte, öffentliche Verkehrsmittel, Flugzeuge, Restaurants, Bars, Cafes, Kirchen, Hotels, öffentliche Gebäude, Eigenheime, Krankenhäuser und/oder andere medizinische oder Pflegeeinrichtungen sein oder umfassen.

Eine "Umweltgröße" kann eine Feuchtigkeit, eine relative Feuchtigkeit, eine Temperatur, ein Luftdruck, eine Feinstaubkonzentration, eine Konzentration eines organischen Elements oder Mikroorganismus, beispielsweise eines Pilzes oder Schimmelpilzes und/oder eine Konzentration eines bestimmten Elements, Gases oder einer bestimmten Gasmischung, beispielsweise eine CO- und/oder eine CO2-Konzentration, einer das Sensorsystem, insbesondere dessen Erfassungseinrichtung, umgebenden Atmosphäre oder Luft sein.

In manchen Ausführungsformen ist das erfindungsgemäße Sensorsystem vorgesehen, ausgestaltet und/oder konfiguriert zur Durchführung des erfindungsgemäßen Verfahrens.

In einigen Ausführungsformen weist das Sensorsystem wenigstens zwei oder mehr Erfassungseinrichtungen und/oder Kommunikationsschnittstellen auf.

Wenn hierin von "programmiert" oder "konfiguriert" oder "ausgebildet" die Rede ist, so ist auch offenbart, diese Begriffe gegeneinander auszutauschen.

Wenn hierin von "programmiert" oder "konfiguriert" oder "ausgebildet" die Rede ist, so ist auch offenbart, dass die betreffende Vorrichtung oder Einrichtung in einigen Ausführungsformen zum automatischen Ausführen von Schritten, zu welchen sie programmiert oder konfiguriert ist, eingerichtet ist.

Wenn hierin ein Schritt oder eine Handlung offenbart ist, so ist auch offenbart, dass jeweils wenigstens eine Vorrichtung oder Einrichtung des Sensorsystems in manchen Ausführungsformen zum Ausführen des Schritts oder der Handlung programmiert oder konfiguriert ist.

Wenn hierin von "Werte einer Umweltgröße berücksichtigen" die Rede ist, so ist auch offenbart, dass ein oder mehrere Werte der Umweltgröße umfasst sind und/oder wenigstens, gegebenenfalls mit anderen Werten, Parametern, Wertebereichen, Variablen und/oder anderweitige Werten und/oder Daten, insbesondere Messwerten, vorzugsweise von weiteren Umweltgrößen, verarbeitet, beispielsweise verglichen, werden.

In manchen Ausführungsformen umfasst das Ausgangssignal wenigstens einen ermittelten Wert der wenigstens einen Umweltgröße.

Alternativ oder ergänzend basiert in bestimmten Ausführungsformen das Ausgangssignal wenigstens auf ermittelten Werten der wenigstens einen Umweltgröße.

Das Ausgangssignal kann in manchen Ausführungsformen eine Auswertung oder andere Weiterverarbeitung der ermittelten Werte der wenigstens einen Umweltgröße sein, darstellen, berücksichtigen und/oder umfassen. Insbesondere kann der ermittelte Wert der wenigstens einen Umweltgröße in ein Farbsignal kodiert oder konvertiert werden. Beispielsweise kann das Unter- oder Überschreiten eines bestimmten Werts oder Wertebereichs des ermittelten Werts der wenigstens einen Umweltgröße farblich unterschiedlich und/oder zahlenmäßig wiedergeben werden. Beispielsweise kann das Ausgangssignal zur Steuerung einer Ampel- und/oder Alarmanzeige verwendet werden. Beispielsweise kann die Farbe Grün auf eine gute Luftqualität, z. B. eine niedrige CO2-Konzentration, die Farbe Gelb oder Orange auf eine mittelmäßige Luftqualität und die Farbe Rot auf eine schlechte und/oder potenziell gesundheitsgefährdende Luftqualität hinweisen.

Das Ausgangssignal kann in bestimmten Ausführungsformen alternativ oder ergänzend Verhaltenshinweise umfassen, welche vorzugsweise wenigstens auf den ermittelten Werten der wenigstens einen Umweltgröße und/oder deren Entwicklung basieren. Ein Verhaltenshinweis kann beispielsweise die Aufforderung beinhalten, eine Be- oder Durchlüftung durchzuführen. Der Verhaltenshinweis kann dem Anwender beispielsweise durch eine visuelle, z. B. Text oder Farbsignal, akustische, z. B. Alarmton, und/oder haptische, z. B. Vibration, Ausgabe kommuniziert werden.

In einigen Ausführungsformen kann das Ausgangssignal und/oder die Auswertung der ermittelten Werte der wenigstens einen Umweltgröße die Berücksichtigung anderer Werte umfassen und/oder berücksichtigen. Andere Werte können insbesondere eine außerhalb des Raumes in welchem sich die Erfassungseinrichtung befindet herrschende äußere Temperatur und/oder Luftfeuchtigkeit, vorzugsweise relative Luftfeuchtigkeit, Grenzwerte, Wertebereiche, festgesetzte und/oder vordefinierte Werte, beispielsweise Standardwerte, insbesondere von Umweltgrößen, Umweltdaten und/oder Umweltparameter sein oder diese umfassen.

Umweltdaten können insbesondere vordefinierte, voreingestellte, festgesetzte und/oder vorab ermittelten Daten, insbesondere Werte, sein oder umfassen. Umweltdaten können, insbesondere von einem Nutzer und/oder einer Umweltdatenerfassungseinrichtung festgelegt oder verändert werden und/oder Standarddaten sein oder umfassen, welche als Standardeinstellung, beispielsweise ab Werk, voreingestellt, vorliegen und/oder gespeichert sind. Umweltdaten können insbesondere eine Personenzahl, eine Raumfläche, ein Raumvolumen, eine Temperatur, eine Luftfeuchtigkeit oder eine relative Luftfeuchtigkeit, ein Luftdruck sein, diesen entsprechen und/oder diese umfassen. Umweltdaten können insbesondere Zahlen, Grenzwerte und/oder Wertbereiche sein, solche umfassen und/oder solchen entsprechen. Umweltdaten können abrufbare Daten sein, welche beispielsweise in einer Datenbank und/oder einem Datenspeicher vorliegen.

"Sensordaten" können insbesondere Messdaten sein, welche vorzugsweise mittels einer Erfassungseinrichtung, beispielsweise einem Sensor oder Sensormodul, erfasst worden sind oder werden.

Umweltdaten und/oder Sensordaten können früher ermittelte Sensordaten und/oder Umweltdaten sein und/oder solche umfassen.

Ein "Ermitteln" oder Bestimmen, insbesondere von Daten und/oder Werten, kann ein Untersuchen einer Existenz oder Nicht-Existenz, ein Erfassen, ein Messen, ein Auswerten, ein Verarbeiten, ein Vergleichen, ein Schätzen, Einschätzen oder Abschätzen, ein Erschließen, ein Berechnen, ein Erhalten, ein Erreichen und/oder ein Erkennen sein oder umfassen.

In einigen Ausführungsformen weist die Erfassungseinrichtung wenigstens zwei oder mehr Sensoren, Sensormodule und/oder weitere Module auf.

In manchen Ausführungsformen kann wenigstens ein Sensormodul insbesondere mehrere Sensoren und/oder eine Sensoranordnung auffassen. Ein, manche oder alle Sensor(en) eines Sensormoduls oder einer Sensoranordnung können ausgebildet und/oder konfiguriert sein, um eine gleiche Umweltgröße (zum Beispiel eine Temperatur) oder mehrere verschiedene Umweltgrößen (zum Beispiel eine Temperatur und eine CO2-Konzentration) zu erfassen, zu bestimmen und/oder zu messen.

In einigen Ausführungsformen kann wenigstens ein Sensor, ein Sensormodul und/oder eine Sensoranordnung als integraler Teil der Erfassungseinrichtung ausgebildet sein.

In manchen Ausführungsformen kann wenigstens ein Sensor, ein Sensormodul und/oder eine Sensoranordnung, beispielsweise modular, insbesondere als ein lösbarer Teil der Erfassungseinrichtung, ausgebildet sein.

In einigen Ausführungsformen kann wenigstens ein Sensor und/oder ein Sensormodul und/oder eine Sensoranordnung derart ausgebildet sein, dass ein Austausch oder ein Ersetzen von einzelnen Sensoren und/oder einem einzelnen Modul möglich und/oder besonders einfach gestaltet ist. Beispielsweise kann die Erfassungseinrichtung wenigstens eine erste Verbindungseinrichtung aufweisen, welche mit einer zweiten Verbindungseinrichtung wenigstens eines Sensors, eines Sensormoduls oder wenigstens eines weiteren Moduls, beispielsweise eines Datenspeichers, lösbar verbindbar ist. Optional kann die Erfassungseinrichtung eine, zwei oder weitere erste Verbindungseinrichtungen, beispielsweise Steckplätze, für entsprechende zweite Verbindungseinrichtungen, beispielsweise Steckvorrichtungen, aufweisen. Zudem kann wenigstens ein, manche oder alle Sensor(en), Sensormodul(e) und/oder Sensoranordnung(en) lösbar, ein- und/oder austeckbar mit der Erfassungseinrichtung verbunden oder verbindbar sein, sodass ein jeweiliges Austauchen und/oder Ersetzen entsprechend ermöglicht wird. Ein, manche oder alle Sensor(en), Sensormodul(e) und/oder weiteren Modul(e) können hierzu beispielsweise jeweils eine entsprechende Verbindungsvorrichtung, beispielsweise eine Steckvorrichtung aufweisen.

In einigen Ausführungsformen kann die Erfassungseinrichtung wenigstens einen CO2-Sensor, ein CO2-Sensormodul und/oder eine CO2-Sensoranordnung aufweisen, wobei beispielsweise eine CO2-Konzentration der umgebenden Atmosphäre oder Luft mittels Infrarotabsorption und/oder mittels eines photo-akustischen Verfahrens ermittelt wird. In bestimmten Ausführungsformen ist der CO2-Sensor ein photo-akustischer Sensor oder umfasst einen solchen.

In manchen Ausführungsformen kann die Erfassungseinrichtung wenigstens einen Anwesenheits- und/oder Bewegungssensor umfassen und/oder mit einem solchen in Datenverbindung stehen.

In bestimmten Ausführungsformen erfolgt die Ermittlung der wenigstens einen Umweltgröße und/oder die Erzeugung eines Ausgangssignals in Echtzeit.

Wenn hierin von "Echtzeit" die Rede ist, so kann insbesondere eine simultan zur Realität ablaufende Zeit verstanden werden. Es kann als eine vorgegebene oder vordefinierte Zeit verstanden werden, insbesondere eine Zeit kleiner als eine Minute, welche beispielsweise als die Zeit definiert ist, die das erfindungsgemäße System in der Realität verbrauchen darf, um ein Ausgangssignal zu erzeugen.

In manchen Ausführungsformen kann die Erfassungseinrichtung wenigstens einen Sensor, ein Sensormodul und/oder eine Sensoranordnung aufweisen, welche dazu ausgebildet und/oder konfiguriert sind, den Wert wenigstens einer Umweltgröße insbesondere der umgebenden Atmosphäre oder Luft mittels jeweils bekannter Verfahren zu ermitteln.

In bestimmten Ausführungsformen kann das Sensorsystem und/oder die Erfassungseinrichtung wenigstens eine Verbindungsleitung umfassen.

In einigen Ausführungsformen kann die Verbindungsleitung insbesondere eine Kommunikationsleitung und/oder eine Versorgungsleitung, insbesondere eine Energie-, Strom- und/oder Spannungsversorgungsleitung, sein oder wenigstens eine solche umfassen.

In manchen Ausführungsformen kann wenigstens eine Verbindungsleitung als integraler oder lösbarer Teil der Erfassungseinrichtung ausgebildet sein und/oder wenigstens von dieser umfasst sein.

In einigen Ausführungsformen kann eine Verbindungsleitung wenigstens einen Anschluss, beispielsweise einen Stecker oder eine Buchse, zum Verbinden mit wenigstens einem Anschluss der Erfassungseinrichtung aufweisen. Alternativ oder ergänzend weist die Verbindungsleitung wenigstens einen Anschluss, beispielsweise einen Stecker oder eine Buchse, zum Verbinden mit wenigstens einem Energie-, Strom- und/oder Spannungsversorger und/oder wenigstens einen Anschluss zum Verbinden mit wenigstens einer externen Vorrichtung und/oder Benutzerschnittstelle auf.

Wenn hierin von einem "Anschluss" die Rede ist, so kann hierunter insbesondere ein Stecker oder eine Buchse verstanden werden. Insbesondere kann darunter ein USB-A, USB-C, Apple Lightning, USB-PD, einen Mini- oder Micro-USB, eine Apple 30-Pol-Verbindung, ein DisplayPort und/oder ein HDMI-Anschluss verstanden werden. Eine Verbindungsleitung kann jede beliebige Anschluss-Kombination aus den oben genannten Anschlüssen aufweisen. Die Erfassungseinrichtung und/oder externe Einrichtungen können wenigstens einen dieser Anschlüsse aufweisen.

In einigen Ausführungsformen kann das Sensorsystem wenigstens einen Mehrfach-Adapter umfassen.

Eine "externe Vorrichtung" kann insbesondere Ausgabemedien wie beispielsweise eine Anzeige, ein Rechner, ein Kommunikationsgerät, insbesondere ein externes tragbares Gerät wie beispielsweise ein Tablet oder ein mobiles Telefongerät sein und/oder wenigstens umfassen. Eine "externe Vorrichtung" kann eine, vorzugsweise steuerbare, Einrichtung oder Vorrichtung sein und/oder wenigstens umfassen, welche beispielsweise Einfluss auf wenigstens eine Umweltgröße nehmen kann, beispielsweise ein Klimagerät, eine Klimaanlage, ein Befeuchtungsgerät, eine Heizung, eine Lüftungseinrichtung und/oder dergleichen.

Eine "Kommunikationsschnittstelle" kann eine Übergangsstelle zwischen verschiedenen Komponenten des Sensorsystems bezeichnen, insbesondere zwischen der Erfassungseinrichtung und weiteren Elementen des Sensorsystems, beispielsweise zwischen der Erfassungseinrichtung und einer Ausgabe-, Anzeigeeinrichtung, Betätigungs- und/oder EingabeEinrichtung, über die ein Datenaustausch und/oder eine Datenverarbeitung realisiert werden. Eine "Kommunikationsschnittstelle" kann eine Übergangsstelle zwischen verschiedenen Komponenten des Sensorsystems und wenigstens einer externen Einrichtung und/oder Vorrichtung sein.

In einigen Ausführungsformen weist das Sensorsystem und/oder die Erfassungseinrichtung wenigstens eine, vorzugsweise bilaterale, Kommunikationsschnittstelle auf. Mit einer bilateralen Kommunikationsschnittstelle können insbesondere Daten in beide Richtungen zwischen verschiedenen Komponenten des Sensorsystems und/oder zwischen verschiedenen Komponenten des Sensorsystems und wenigstens einer externen Einrichtung und/oder Vorrichtung ausgetaucht und/oder übermittelt werden.

In manchen Ausführungsformen umfasst die Kommunikationsschnittstelle wenigstens eine drahtlose Kommunikationsverbindung, insbesondere eine Datenkommunikationsverbindung, weist wenigstens eine solche auf oder ist mit wenigstens einer solchen in Datenverbindung. Eine drahtlose Verbindung kann beispielsweise eine kabellose Verbindung über Bluetooth, WirelessHART, WSAN oder Wireless LAN sein, insbesondere eine Verbindung mit Internet, mit einem Intranet, mit einem WLAN-fähiges Gerät, mit einem Router, der ein Drahtlosnetzwerk zur Verfügung stellt und/oder mit einem Funknetz, beispielsweise einem Mobilfunknetz.

Unter "Kommunikationsverbindung" kann insbesondere eine Verbindung verstanden werden, über welche die Übertragung und/oder der Austausch von Nachrichten, Daten, beispielsweise Messdaten, Standardwerten, Umweltdaten und/oder Parametern, insbesondere Umweltparametern, zwischen wenigstens einer Sendeeinrichtung und einer oder mehreren Empfangseinrichtungen, beispielsweise einer Benutzerschnittstelle, einer Anzeigeeinrichtung, einer Eingabeeinrichtung, einer externen Vorrichtung oder Einrichtung und/oder einer Erfassungseinrichtung, vorgesehen, stattfindet und/oder stattfinden kann.

Unter "drahtloser Kommunikationsverbindung" kann insbesondere eine Kommunikationsverbindung verstanden werden, bei welcher die Datenübertragung über eine nicht kabelgebundene, also drahtlose, Kommunikationsschnittstelle stattfindet, beispielsweise können Daten über eine Cloud synchronisiert oder übertragen werden.

Wenn hierin von einer Signal- oder Kommunikationsverbindung insbesondere zweier Komponenten die Rede ist, so kann hierunter eine im Gebrauch bestehende Verbindung zu verstehen sein. Ebenso kann hierunter zu verstehen sein, dass eine Vorbereitung zu einer solchen (kabelgebundenen, kabellosen oder auf andere Weise umgesetzten) Signalkommunikation besteht, beispielsweise durch eine Kopplung beider Komponenten, etwa mittels pairing, usw.

Unter pairing versteht man einen Prozess, der im Zusammenhang mit Rechnernetzwerken erfolgt, um eine anfängliche Verknüpfung zwischen Rechnereinheiten zum Zwecke der Kommunikation herzustellen. Das bekannteste Beispiel hierfür ist das Herstellen einer Bluetooth-Verbindung, mittels welcher verschiedene Einrichtungen (z. B. Smartphone, Kopfhörer) miteinander verbunden werden. Pairing wird gelegentlich auch als bonding bezeichnet.

In einigen Ausführungsformen ist oder umfasst die Kommunikationsschnittstelle wenigstens eine Kommunikationsleitung, insbesondere wenigstens eine Datenleitung, zum Beispiel ein Datenkabel. Eine Kommunikationsleitung kann insbesondere mehrere Übertragungskanäle umfassen.

In bestimmten Ausführungsformen kann eine Kommunikationsleitung wenigstens eine Energieversorgungsleitung umfassen.

In bestimmten Ausführungsformen weist das Sensorsystem, insbesondere dessen Erfassungseinrichtung, wenigstens eine Energie-, Strom- und/oder Spannungsquelle oder Energie-, Strom- und/oder Spannungsversorger auf und/oder ist wenigstens mittels einer externen Energie-, Strom- und/oder Spannungsquelle oder Energie-, Strom- und/oder Spannungsversorger aufladbar und/oder wenigstens verbindbar, vorzugsweise mittels eines, vorzugsweise abnehmbaren, Versorgungskabels. Alternativ oder ergänzend kann das Sensorsystem, insbesondere dessen Erfassungseinrichtung, induktiv aufladbar sein, beziehungsweise dazu ausgebildet sein, durch induktive Stromübertragung aufgeladen zu werden. Alternativ oder ergänzend kann das Sensorsystem, insbesondere dessen Erfassungseinrichtung, einen Steckdosenstecker umfassen, um direkt an einem Stromnetz angeschlossen zu werden.

Eine "Energie-, Strom- und/oder Spannungsquelle" oder ein "Energie-, Strom- und/oder Spannungsversorger" kann beispielsweise ein Energiespeicher, eine Batterie, oder ein, vorzugsweise wiederaufladbarer, Akku sein.

Eine "externe Energie-, Strom- und/oder Spannungsquelle" oder ein "externer Energie-, Strom- und/oder Spannungsversorger" kann beispielsweise ein vorhandenes Stromnetz, ein mit einem Stromnetz verbundenes Netzteil, ein Energiespeicher, eine Batterie, ein, vorzugsweise wiederaufladbarer, Akku, beispielsweise eine Powerbank, und/oder ein sonstiges elektrisches Gerät mit eigener Energieversorgung, beispielsweise ein Laptop, ein Rechner oder ein Mobiltelefon sein.

Mittels der Energie-, Strom- und/oder Spannungsquelle bzw. der Verbindung zu einer externen Energie-, Strom- und/oder Spannungsquelle werden alle oder manche der verschiedenen Komponente des Sensorsystems, insbesondere dessen Erfassungseinrichtung, ein, manche, oder alle Sensor(en), Module, und/oder Einrichtungen des Sensorsystems, mit Energie, Strom und/oder Spannung versorgt, so dass sie jeweils betrieben, geregelt und/oder gesteuert werden können.

In einigen Ausführungsformen weist das Sensorsystem und/oder die Erfassungseinrichtung wenigstens ein Netzgerät auf, beispielsweise einen Transformator und/oder ein Netzteil, das eine Netzspannung auf die von der Erfassungseinrichtung und/oder den Sensor(en), Modulen, und/oder Einrichtungen des Sensorsystems benötigte Spannungsstärke vermindert. Alternativ oder ergänzend kann ein solches Netzgerät mittels einer Verbindungsleitung mit dem Sensorsystem und/oder mit dessen Erfassungseinrichtung Sensor(en), Modulen, und/oder Einrichtungen vorzugsweise trennbar, verbunden sein oder verbindbar sein. Das Netzgerät kann zum Aufladen eines Energiespeichers, beispielsweise eines Akkus, des Sensorsystems und/oder dessen Erfassungseinrichtung, vorzugsweise zeitweise, vorgesehen und/oder ausgebildet sein.

In manchen Ausführungsformen kann die Kommunikationsschnittstelle wenigstens eine Sende- und/oder Empfangseinrichtung aufweisen oder daraus bestehen, und/oder kann mit wenigstens einer Sende- und/oder Empfangseinrichtung in Signalverbindung, insbesondere in Datenverbindung, stehen.

In einigen Ausführungsformen kann das Sensorsystem, insbesondere dessen Erfassungseinrichtung, wenigstens eine Sendeeinrichtung, welche als Sendemodul ausgebildet ist, umfassen. Das Sendemodul kann wenigstens eine Antenne aufweisen. Das Sendemodul kann dazu ausgebildet und/oder dazu programmiert sein, von den Sensoren ermittelten Daten an eine Empfangseinrichtung oder an ein Empfangsmodul, vorzugsweise über eine drahtlose Kommunikationsverbindung, insbesondere Datenkommunikationsverbindung, beispielsweise über Bluetooth, zu übermitteln.

In bestimmten Ausführungsformen kann eine Sende- und/oder Empfangseinrichtung beispielsweise wenigstens eine Antenne aufweisen. Eine Antenne kann beispielsweise in der Erfassungseinrichtung angeordnet und/oder ausgebildet sein, und/oder Teil der Erfassungseinrichtung sein.

In manchen Ausführungsformen kann das Sensorsystem, insbesondere dessen Erfassungseinrichtung, wenigstens eine Empfangseinrichtung oder ein Empfangsmodul umfassen. Die Empfangseinrichtung oder das Empfangsmodul kann dazu ausgebildet und/oder dazu programmiert sein, Eingaben, Parameter, insbesondere Umweltparameter und/oder Daten, beispielsweise welche die zur Erzeugung eines Ausgangssignals und/oder zum Steuern und/oder Regeln des Sensorsystems, der Erfassungseinrichtung, einer Steuer- und/oder Regeleinrichtung des Sensorsystems und/oder wenigstens eines Sensors, notwendig, vorgesehen und/oder geeignet sind, vorzugsweise über eine drahtlose Kommunikationsverbindung, beispielsweise über Bluetooth, zu empfangen und an beteiligte Komponenten zu übermitteln.

In einigen Ausführungsformen weist das Sensorsystem und/oder die Erfassungseinrichtung wenigstens einen Datenspeicher auf. Der Datenspeicher kann insbesondere zum Speichern von Daten der Erfassungseinrichtung, insbesondere Messdaten der Sensoren und/oder Sensormodule, und/oder von weiteren Daten, Parameter und/oder Variablen, insbesondere vordefinierte, eingegebene und/oder Standardwerte, ausgebildet und/oder konfiguriert sein. Der Datenspeicher kann beispielsweise Daten umfassen, einspeisen oder bereitstellen, welche für die Erzeugung wenigstens eines Ausgangssignals relevant und/oder benötigt sind oder werden.

In manchen Ausführungsformen kann der Datenspeicher als integraler oder lösbarer Teil der Erfassungseinrichtung ausgebildet und/oder konfiguriert sein.

In manchen Ausführungsformen kann der Datenspeicher ein externer Datenspeicher sein und/oder einen solchen umfassen, beispielsweise ein Cloud-Speicher und/oder ein Datenspeicher einer externen Vorrichtung, wie beispielsweise Ausgabemedien wie ein Rechner, ein Tablet oder ein mobiles Telefongerät, sein.

In einigen Ausführungsformen ist der Datenspeicher modular ausgebildet, beispielsweise als USB-Massenspeicher, beispielsweise als USB-Stick und/oder als SD-Karte.

In manchen Ausführungsformen umfasst die Erfassungseinrichtung wenigstens ein Gehäuse.

In einigen Ausführungsformen weist das Gehäuse wenigstens einen Anschluss für eine Verbindungsleitung, beispielsweise für eine Kommunikationsleitung und/oder für eine Versorgungsleitung, auf.

In bestimmten Ausführungsformen ist das Gehäuse leicht integrierbar und/oder weist eine kleine und/oder kompakte Bauform auf. Somit kann die Erfassungseinrichtung leicht transportiert, abgestellt und/oder als mobiles Gerät verwendet werden.

In einigen Ausführungsformen weist die Erfassungseinrichtung, insbesondere das Gehäuse der Erfassungseinrichtung, wenigstens eine Dimension, z. B. Höhe und/oder Breite, welche kleiner oder gleich 5 cm, vorzugsweise kleiner oder gleich 36 mm, ist und/oder wenigstens eine Dimension, z. B. Dicke, welche kleiner oder gleich 1 cm, vorzugsweise kleiner oder gleich 0,5 cm ist, auf.

In manchen Ausführungsformen hat die Erfassungseinrichtung, insbesondere das Gehäuse der Erfassungseinrichtung, einen Durchmesser bis 25,75 mm und eine Dicke bis 2,20 mm, ist also nicht größer als eine 2-Euro-Münze, oder gleich groß.

In bestimmten Ausführungsformen weist die Erfassungseinrichtung, insbesondere das Gehäuse der Erfassungseinrichtung, einen Durchmesser, eine Fläche, eine Größe und/oder ein Volumen auf, welche(r)(s) kleiner, gleich, 1,5-mal größer, 2-, 2,5-, 3-, 3,5- oder 4-mal größer als die, der oder das einer 2-Euro-Münze.

In manchen Ausführungsformen kann das Gehäuse ein Innenraum und/oder einzelne Teile oder Innenräume der Erfassungseinrichtung umgeben. Insbesondere kann das Gehäuse, oder Teile hiervon, einen einzelnen Sensor oder mehrere Sensoren umgeben. Das Gehäuse kann alle Komponenten einer Erfassungseinrichtung umgeben, so dass die Komponenten der Erfassungseinrichtung wie beispielsweise Sensoren, Sensormodule, weitere Module und/oder Einrichtungen der Erfassungseinrichtung vorzugsweise in einem Innenraum des Gehäuses angeordnet, vorhanden, ausgebildet und/oder eingebaut sind. Das Gehäuse kann aus mehreren Teilen ausgebildet sein, welche miteinander und/oder mit einem Äußeren des Gehäuses, insbesondere luftdicht oder luftdurchlässig, verbunden sind.

In einigen Ausführungsformen können Teile eines mehrteiligen Gehäuses lösbar voneinander verbunden, beispielsweise miteinander verschraubt und/oder verklemmt sein.

In manchen Ausführungsformen können eine oder mehrere Dichtungen zum Abdichten eines Innenraums oder der Innenräume der Erfassungseinrichtung von einem Außenraum der Erfassungseinrichtung und/oder zum Abdichten der Innenräume untereinander vorgesehen und/oder vorhanden sein.

In bestimmten Ausführungsformen kann das Gehäuse aus nicht voneinander lösbaren, beispielsweise miteinander verlöteten und/oder verschweißten Gehäuseteile ausgebildet sein.

In einigen Ausführungsformen kann das Gehäuse wenigstens eine zu einem Außenraum des Gehäuses und/oder zu einem Außenraum der Erfassungseinrichtung geöffnete Öffnung, oder einen Zugang aufweisen.

In manchen Ausführungsformen kann eine Öffnung oder ein Zugang dazu ausgebildet und/oder konfiguriert sein, ein Eindringen von Flüssigkeit, beispielsweise Wasser, zu verhindern. Beispielsweise kann die Öffnung oder der Durchgang mit einer luftdurchlässigen, aber wasserabweisender Membrane ausgebildet oder ausgerüstet sein. Alternativ oder ergänzend kann das Gehäuse wenigstens eine Dichtung aufweisen und/oder derart ausgebildet und/oder zusammengesetzt sein, dass ein Eindringen von Flüssigkeit, insbesondere von der Seite und/oder von oben, insbesondere in einer vorgesehenen Betriebsausrichtung der Erfassungseinrichtung, vermindert oder verhindert wird. Beispielsweise kann ein Teil des Gehäuses einen entsprechenden Vorsprung aufweisen, welcher wenigstens eine Öffnung seitlich und/oder oben abdeckt. Alternativ oder ergänzend kann wenigstens eine Außenfläche des Gehäuses beispielsweise eine nach außen gewölbte Fläche und/oder eine wasserabweisende Oberfläche aufweisen.

In einigen Ausführungsformen kann das Gehäuse und/oder Teile des Gehäuses aus Metall, Glas, recyclebaren Materialien, beispielsweise Holz, Papier oder Kartonagen, und/oder aus Kunststoff oder Kunststoffmischungen gefertigt und/oder zumindest teilweise ausgebildet sein. Das Gehäuse und/oder Teile des Gehäuses können transparent, teilweise transparent und/oder zumindest teilweise transluzent sein und/oder ausgebildet sein.

In manchen Ausführungsformen kann das Gehäuse eine Halte- und/oder Hängevorrichtung, beispielsweise einen Haken oder einen Steh-Fuß, aufweisen. Eine Halte- und/oder Hängevorrichtung kann vorzugsweise einklappbar und/oder abnehmbar ausgebildet sein.

In bestimmten Ausführungsformen weist das Sensorsystem und/oder die Erfassungseinrichtung, insbesondere das Gehäuse der Erfassungseinrichtung, wenigstens eine Benutzerschnittstelle auf. Eine Benutzerschnittstelle kann insbesondere wenigstens eine Ausgabe-, Alarm-, Anzeige-, Betätigungs- und/oder Eingabeeinrichtung oder -vorrichtung sein oder aufweisen. Alternativ oder ergänzend kann eine Benutzerschnittstelle insbesondere wenigstens mit wenigstens einer solchen Vorrichtung in, vorzugsweise drahtloser, Daten- und/oder Kommunikationsverbindung stehen oder, wenigstens zeitweise, verbindbar sein.

Wenn hierin von "Benutzerschnittstelle", "Benutzervorrichtung", "Benutzereinrichtung" die Rede ist, so ist auch offenbart, diese Begriffe gegeneinander auszutauschen.

In manchen Ausführungsformen kann eine Benutzerschnittstelle insbesondere wenigstens eine Benutzeroberfläche, insbesondere eine Anzeige- und/oder Ausgabeoberfläche aufweisen, umfassen und/oder mit einer solchen in Verbindung, insbesondere in Datenverbindung, vorzugsweise drahtlos, stehen.

In bestimmten Ausführungsformen kann die Benutzerschnittstelle und/oder die Benutzeroberfläche insbesondere die Benutzeroberfläche wenigstens einer Ausgabe-, Alarm-, Anzeige-, Betätigungs-, Eingabeeinrichtung und/oder wenigstens einer externen Einrichtung und/oder Vorrichtung sein, umfassen und/oder auf diese darstellbar und/oder ausführbar sein.

In manchen Ausführungsformen kann die Benutzerschnittstelle und/oder die Benutzeroberfläche insbesondere eine Software und/oder ein GUI ("Graphical User Interface") aufweisen, welche(s) auf einer externen Vorrichtung ausgeführt wird oder werden kann, beispielsweise eine App und/oder ein Computerprogramm. Die Software und/oder das GUI kann insbesondere für ihre/seine Ausführung auf verschiedenen Plattformen oder mit verschiedenen (Betriebs-)Systemen, beispielsweise Windows, Android, iOS, Linux vorgesehen, konfiguriert und/oder programmiert sein. Die Benutzerschnittstelle und/oder die Benutzeroberfläche sind oder stehen vorzugsweise in, insbesondere drahtloser, Datenverbindung mit der Erfassungseinrichtung und/oder mit den jeweiligen Einrichtungen des Sensorsystems und/oder der Erfassungseinrichtung.

In manchen Ausführungsformen ist die Benutzerschnittstelle und/oder die Benutzeroberfläche eine Software und/oder ein GUI, welche(s) vorzugsweise auf einer externen Vorrichtung, beispielsweise einem Handy, Rechner und/oder Laptop, ausgeführt wird und/oder ausführbar und/oder bedienbar ist. In einigen Ausführungsformen kann die Ausgabeeinrichtung eine Einrichtung zum Ausgeben wenigstens eines Ausgangssignals sein. Die Ausgabeeinrichtung kann ausgebildet, konfiguriert und/oder programmiert sein, zumindest ein Ausgangsignal wenigstens auf Basis der mit der Erfassungseinrichtung ermittelten Werte oder Daten zu erzeugen, bereitzustellen, auszuwerten und/oder zu verarbeiten. Die Einrichtung zum Ausgeben wenigstens eines Ausgangssignals kann insbesondere ausgebildet, konfiguriert und/oder programmiert sein, zumindest ein Ausgangsignal wenigstens auf Basis der mit der Erfassungseinrichtung ermittelten Daten zu erzeugen.

In manchen Ausführungsformen kann die Ausgabeeinrichtung ausgebildet, konfiguriert und/oder programmiert sein, ein Alarmsignal auszugeben, beispielsweise ein akustisches und/oder optisches Signal.

In einigen Ausführungsformen kann die Ausgabeeinrichtung und/oder Anzeigeeinrichtung ausgebildet, konfiguriert und/oder programmiert sein, ein Ausgangssignal zu erzeugen, welcher geeignet und/oder vorgesehen ist, angezeigt und/oder dargestellt zu werden, beispielsweise auf einem Bildschirm oder Display, insbesondere einer externen Vorrichtung.

In manchen Ausführungsformen kann insbesondere die Erfassungseinrichtung wenigstens eine Betätigungseinrichtung und/oder eine Eingabeeinrichtung aufweisen.

In einigen Ausführungsformen sind die Betätigungseinrichtung und/oder die Eingabeeinrichtung als Teil des Gehäuses der Erfassungseinrichtung ausgebildet. Beispielsweise können die Betätigungseinrichtung und/oder die Eingabeeinrichtung als Display mit verschiedensten Schaltflächen, beispielsweise einem Drehrad und/oder Druckknopf und/oder Druckschalter

(Button) ausgebildet sein. Es kann mittels der Betätigungseinrichtung und/oder der Eingabeeinrichtung vorgesehen und/oder möglich sein, Daten an das Sensorsystem zu übermitteln, für dieses einzugeben und/oder zu ändern, die Erfassungseinrichtung ein und/oder auszuschalten und dergleichen.

In manchen Ausführungsformen können die Ausgabe-, Alarm-, Anzeige-, Betätigungs- und/oder die Eingabeeinrichtung jeweils einzeln oder zusammen Teil der Erfassungseinrichtung und/oder des Gehäuses der Erfassungseinrichtung sein. Die Ausgabe-, Alarm-, Anzeige-, Betätigungs- und/oder die Eingabeeinrichtung können paarweise oder alle zusammen eine einzige Einrichtung bilden.

In einigen Ausführungsformen können die Ausgabe-, Alarm- und/oder Anzeigeeinrichtung, die Betätigungseinrichtung und/oder die Eingabeeinrichtung Teile einer externen Einrichtung sein und lediglich in, vorzugsweise drahtlose, Datenverbindung mit der Erfassungseinrichtung sein.

In bestimmten Ausführungsformen umfasst das Ausgangssignal wenigstens eine Aussage über die mit der Erfassungseinrichtung ermittelte Umweltgröße. Das Ausgangssignal kann vorzugsweise eine Handlungsempfehlung auf Basis der mit der Erfassungseinrichtung ermittelte Umweltgröße umfassen und/oder erzeugen.

In einigen Ausführungsformen weist das Sensorsystem, insbesondere dessen Erfassungseinrichtung, wenigstens einen Taktgeber auf. Der Taktgeber kann insbesondere zum Synchronisieren und/oder Steuern der Datenabfragen der verschiedenen Systemkomponenten des Sensorsystems, insbesondere der Erfassungseinrichtung, dienen.

In manchen Ausführungsformen weist das Sensorsystem und/oder dessen Erfassungseinrichtung wenigstens eine Steuer- und/oder Regeleinrichtung zum Steuern und/oder Regeln des Sensorsystems auf.

In einigen Ausführungsformen kann die Steuer- und/oder Regeleinrichtung dazu ausgebildet und/oder programmiert sein, ein Verfahren zur Überwachung wenigstens einer Umweltgröße auszuführen und/oder das Sensorsystem, insbesondere dessen Erfassungseinrichtung, zu regeln und/oder zu steuern.

In manchen Ausführungsformen ist die Steuer- und/oder Regeleinrichtung Teil des Sensorsystems, insbesondere Teil der Erfassungseinrichtung.

In bestimmten Ausführungsformen kann die Steuer- und/oder Regeleinrichtung in Datenverbindung, vorzugsweise in drahtloser Verbindung, mit der Erfassungseinrichtung stehen.

In einigen Ausführungsformen ist die Steuer- und/oder Regeleinrichtung Teil einer externen Einrichtung und steht lediglich in Datenverbindung, vorzugsweise in drahtloser Verbindung, mit der Erfassungseinrichtung.

In manchen Ausführungsformen kann die Steuer- und/oder Regeleinrichtung ausgebildet und/oder programmiert sein, um in Datenverbindung mit wenigstens einer externen Vorrichtung, beispielsweise mit einer Klimaanlage und/oder einem Befeuchtungsgerät zu stehen. Insbesondere kann die Steuer- und/oder Regeleinrichtung ausgebildet und/oder programmiert sein, um, insbesondere auf Basis der von der Erfassungseinrichtung ermittelten Daten und/oder eines Ausgangssignals der Erfassungseinrichtung, diese Geräte zu steuern und/oder zu regeln.

In einigen Ausführungsformen ist die Steuer- und/oder Regeleinrichtung kein Teil der Erfassungseinrichtung. In diesen Ausführungsformen kann die Steuer- und/oder Regeleinrichtung von der Erfassungseinrichtung räumlich getrennt sein und/oder mit dieser nur in, vorzugsweise drahtloser, Daten- und/oder Kommunikationsverbindung stehen.

In manchen Ausführungsformen kann die Steuer- und/oder Regeleinrichtung eine Software und/oder ein GUI, welche(s) vorzugsweise auf einer externen Vorrichtung, beispielsweise einem Handy, Rechner und/oder Laptop, ausgeführt wird und/oder auf dieser ausführbar und/oder bedienbar ist.

In manchen Ausführungsformen weist die Erfassungseinrichtung wenigstens einen CO-Sensor, wenigstens einen CO2-Sensor, wenigstens einen Temperatursensor, wenigstens einen Luftdrucksensor, und/oder wenigstens einen Feuchtigkeitssensor auf.

In einigen Ausführungsformen weist die Erfassungseinrichtung wenigstens einen CO2-Sensor und wenigstens einen Feuchtigkeitssensor auf. Der Feuchtigkeitssensor kann insbesondere dazu ausgebildet und/oder programmierts ein, eine relative Luftfeuchtigkeit der umgebenden Luft zu ermitteln.

In manchen Ausführungsformen weist die Erfassungseinrichtung wenigstens einen CO2-Sensor, wenigstens ein Temperatursensor und wenigstens einen Feuchtigkeitssensor auf.

In einigen Ausführungsformen kann die Erfassungseinrichtung, insbesondere das Gehäuse der Erfassungseinrichtung des Sensorsystems wenigstens eine akustische, optische und/oder haptische Anzeigeeinrichtung aufweisen.

In manchen Ausführungsformen weist die Erfassungseinrichtung wenigstens einen Ein- und/oder Ausschalter auf. Der Zustand der Erfassungseinrichtung, insbesondere, ob die Erfassungseinrichtung in Betrieb ist, kann mittels einer Statusanzeige, welche Teil eines Gehäuses sein kann, darstellbar sein. Beispielsweise kann die Statusanzeige eine LED-Leuchte oder -Anordnung sein oder umfassen.

In einigen Ausführungsformen können die Anzeigeeinrichtung, die Eingabeeinrichtung und/oder die Betätigungseinrichtung können, zusammen oder einzeln, explizit kein Teil der Erfassungseinrichtung sein. Vorzugsweise können diese räumlich getrennt sein. Somit kann beispielsweise eine Erfassungseinrichtung an eine bestimmte Stelle platziert werden, während die Überwachung der Umweltgröße und/oder die Steuerung und/oder Bedienung des Sensorsystems vorteilhaft von einer räumlich getrennten Stelle stattfinden kann.

In einigen Ausführungsformen umfasst das Verfahren die Bereitstellung wenigstens eines erfindungsgemäßen Sensorsystems.

In manchen Ausführungsformen umfasst das Verfahren das Ermitteln wenigstens einer Umweltgröße mittels wenigstens einer Erfassungseinrichtung eines erfindungsgemäßen Sensorsystems.

In einigen Ausführungsformen umfasst das Verfahren die Bereitstellung wenigstens eines erfindungsgemäßen Sensorsystems.

In manchen Ausführungsformen umfasst das Verfahren die Bereitstellung wenigstens einer Ausgabe-, Benutzerschnittstelle und/oder wenigstens einer externen Vorrichtung.

In einigen Ausführungsformen umfasst das Verfahren die Bereitstellung, Eingabe Ermittlung und/oder Übermitteln von Grenzwerten und/oder Wertebereiche für wenigstens eine Umweltgröße und/oder Umweltdaten.

In bestimmten Ausführungsformen umfasst das Verfahren das Bereitstellen, Ermitteln und/oder Übermitteln von vordefinierten und/oder gemessenen Umweltdaten und/oder Umweltparameter.

Beispielsweise kann eine Obergrenze für eine CO2-Konzentration 1000 ppm oder 1200 ppm (parts per million, 10⁻⁶) betragen. Beispielsweise kann eine Untergrenze für eine CO2-Konzentration einen Wert zwischen 400 und 600 ppm betragen. Beispielsweise kann ein Wertebereich für eine CO2-Konzentration 400 bis 1000 ppm betragen.

Beispielsweise kann eine Obergrenze für eine relative Luftfeuchtigkeit 60 % bis 80 % betragen. Beispielsweise kann eine Untergrenze für eine relative Luftfeuchtigkeit Werte unterhalb von 60 %, insbesondere unterhalb von 40 %, betragen. Beispielsweise kann ein Wertebereich für eine relative Luftfeuchtigkeit 40 % bis 60 % betragen.

In bestimmten Ausführungsformen umfasst das Verfahren das Vergleichen des wenigstens einen ermittelten Wertes einer Umweltgröße mit vorher bereitgestellten, ermittelten, vorgegebenen, vordefinierten, festgesetzten und/oder vorab eingegebenen Grenzwerten oder Wertebereichen.

In einigen Ausführungsformen umfasst das Verfahren die Erzeugung oder das Treffen wenigstens einer Aussage, beispielsweise über die Luftqualität des Raumes in welchem sich das Sensorsystem, insbesondere dessen Erfassungseinrichtung, sich befindet.

Eine Aussage kann einen Hinweis oder mehrere Hinweise sein oder umfassen. Ein Hinweis kann eine Handlungsempfehlung und/oder eine Information sein oder umfassen. Eine Handlungsempfehlung kann beispielsweise die Aufforderung sein oder umfassen, einen Belüftungsvorgang einzuleiten. Eine Information kann beispielsweise den derzeitigen Wert einer Umweltgröße sein oder umfassen und/oder eine Feststellung sein oder umfassen, dass eine bestimmte Umweltgröße in einen bestimmten Wertebereich befindet. Beispielsweise kann festgestellt werden, dass eine bestimmte Luftfeuchtigkeit unterschritten wurde, z. B. 40 %, welche zur Austrocknung von Schleimhäuten führen kann.

In einigen Ausführungsformen umfasst das Verfahren die Erzeugung eines Steuerungs- und/oder Regelungssignals für eine externe Vorrichtung, beispielsweise für eine Klimaanlage.

In manchen Ausführungsformen umfasst das Verfahren die Berechnung einer Dauer und/oder einer Frequenz eines geeigneten Belüftungsvorgangs oder Betriebszeit einer externen Vorrichtung, beispielsweise einer Klimaanlage.

In einigen Ausführungsformen umfasst das Verfahren das Steuern und/oder Regeln einer externen Vorrichtung, beispielsweise eines Klimagerätes, wenigstens als Funktion oder unter Berücksichtigung wenigstens eines Ausgangssignals.

In bestimmten Ausführungsformen umfasst das Verfahren die Anpassung einer Messfrequenz (Taktung) als Funktion des Ausgangssignals. Beispielsweise kann eine Messfrequenz erhöht werden, wenn sich gemessene Werte einem Grenzwert nähern.

In manchen Ausführungsformen umfasst das Verfahren die Ermittlung der Raumgröße, insbesondere des Raumvolumens in welchem das Sensorsystem, insbesondere dessen Erfassungseinrichtung, Anwendung findet.

In einigen Ausführungsformen erfolgt die Ermittlung der Raumgröße mit Hilfe von einer bildbasierten Kantenvermessung, einer manuellen Eingabe, einer Datenbank und/oder durch Verwendung einer entsprechenden Software und/oder einem entsprechenden GUI.

In manchen Ausführungsformen umfasst das Verfahren die Ermittlung und/oder Berücksichtigung, ob der Raum in welchem das Sensorsystem, insbesondere dessen Erfassungseinrichtung, Anwendung findet oder sich befindet mit Fenstern, mit einer externen Vorrichtung, wie beispielsweise eine Klimaanlage, ausgerüstet ist und/oder über eine Filtereinrichtung, beispielsweise einen HEPA-Filter verfügt.

In einigen Ausführungsformen umfasst das Verfahren die Ermittlung der Anzahl der sich im Raum befindenden Anzahl der Lebewesen, vorzugsweise samt Aufenthaltsdauer. Hierzu kann beispielsweise wenigstens eine Personenerfassungseinrichtung mit dem erfindungsgemäßen Sensorsystem in Datenverbindung stehen oder verbindbar sein und/oder Teil hiervon sein.

In manchen Ausführungsformen umfasst das Verfahren eine akustische, optische und/oder haptische Wiedergabe und/oder eine Ausgabe wenigstens eines Ausgangssignals. ausgegeben wird.

In einigen Ausführungsformen umfasst das Ausgangsignal die mit der Erfassungseinrichtung gewonnenen Messdaten.

In manchen Ausführungsformen umfasst das Verfahren die Berücksichtigung des Einflusses der Luftfeuchtigkeit oder der relativen Luftfeuchtigkeit auf die potentielle oder geschätzte Halte- oder Verweilzeit von Aerosolen in der Luft, welche insbesondere bei höherer Luftfeuchtigkeit, beispielsweise über 80 % Luftfeuchtigkeit, etwa 20 Mal, also eine oder zwei Größenordnung, höher sein kann.

In manchen Ausführungsformen umfasst wird im Verfahren den Einfluss eines Abstandes einer Quelle, welche eine Umweltgröße verändern kann, berücksichtigt. Dies kann beispielsweise der Abstand einer Person zum verwendeten Sensorsystem, insbesondere zu mindestens einer seiner Erfassungseinrichtungen, sein oder umfassen, wobei die Atmung dieser Person eine CO2-Konzentrationsänderung verursachen kann.

In einigen Ausführungsformen des Sensorsystems weist das Sensorsystem keine im Innenraum des Gehäuses angeordnete Energiequelle auf.

In manchen Ausführungsformen des Sensorsystems weist das Sensorsystem keinen im Innenraum des Gehäuses angeordneten Datenspeicher auf.

In einigen Ausführungsformen des Sensorsystems weist das Gehäuse des Sensorsystems keinen männlichen USB-Stecker auf.

In manchen Ausführungsformen des Sensorsystems weist das Sensorsystem, insbesondere das Gehäuse des Sensorsystems, keine Anzeige, insbesondere keine LED-Ampel oder -Anzeige, insbesondere zum Ausgeben und/oder Anzeigen eines Ausgangssignals auf.

Einige oder alle erfindungsgemäßen Ausführungsformen können einen oder mehrere der oben oder im Folgenden genannten Vorteile aufweisen.

Ein Vorteil der vorliegenden Erfindung kann darin bestehen, ein potenzielles Ansteckungsrisiko zu vermindern oder gar zu eliminieren, indem eine potenzielle Belastung der Raumluft geschlossener Räume mit Viren, z. B. mit Rhinoviren, Influenza-Viren, Sars CoV-2, anderen Corona-Viren und/oder weiteren Viren, wenigstens mittels CO2-Messung ermittelt.

Mittels der vorliegenden Erfindung erhalten Anwender zuverlässige Echtzeit-Hinweise über die Luftqualität ihrer Umgebung und können daraufhin selbst Maßnahmen einleiten, diese (wieder) zu verbessern. Dadurch kann ein beruhigendes Gefühl der Sicherheit vermittelt werden, wodurch psychologischer Stress vermieden werden kann. Dies dürfte vor allem für Eltern, Lehrende, Pflegende, Behandelnde, Angehörige zu Hochrisikogruppen, Personen, die keine Möglichkeit auf Home-Office haben, Wissbegierige, Personen mit einem erhöhten Sicherheitsbedürfnis, Lebensoptimierer, Reisende und/oder Menschen, die in Großstädten leben, von Vorteil sein.

Ein weiterer Vorteil kann in einer Empfehlung für einen bedarfsgerechten Luftaustausch oder Frischluftzufuhr bestehen, wobei diese optisch, akustisch und/oder haptisch kommuniziert, beispielsweise angezeigt werden und/oder veranlasst werden kann.

Die CO2-Konzentration in einem Raum hängt von vielen Faktoren ab, beispielsweise von der Raumgröße, der Anzahl, Art und Aufenthaltsdauer der darin anwesenden Lebewesen, der Temperatur, der Feuchte-, Lüftungs- und Luftströmungsverhältnisse im besagten Raum und so weiter. In manchen Ausführungsformen können solche Umweltparameter vorteilhaft Rechnung getragen werden und/oder diese berücksichtigt werden.

Aerosole können sich deutlich länger in feuchter Luft als in trockener Luft halten ohne zu Boden zu sinken, beispielsweise in einem Bereich von über 80 % relative Luftfeuchte. Dagegen führt eine relative Luftfeuchte von unterhalb 40 % zur Austrocknung der Schleimhäute der anwesenden Personen, welche wiederum die Aufnahme von Erregern begünstigen kann. In manchen Ausführungsformen der vorliegenden Erfindung kann daher die relative Luftfeuchte vorteilhaft zusätzlich berücksichtigt werden, insbesondere bei der Empfehlung für die Dauer und/oder Frequenz eines Luftaustauschs oder einer Frischluftzufuhr.

Ein weiterer Vorteil der vorliegenden Erfindung kann darin bestehen, auf Basis des Ausgangssignals geeignete Belüftungszeitpunkte und/oder eine Belüftungsfrequenz zu ermitteln und diese zu kommunizieren und/oder eine Belüftung, manuell oder automatisch, zu veranlassen. Durch das Lüften werden sowohl eine CO2- als auch eine Aerosolenkonzentration, und somit ein potenzielles Ansteckungsrisiko der anwesenden Personen gesenkt, wodurch deren Sicherheit mittelbar erhöht wird.

Ein weiterer Vorteil der vorliegenden Erfindung kann darin bestehen, insbesondere wenn z. B. eine Klima- und/oder Belüftungsanlage auf Basis des Ausgangssignals gesteuert und/oder geregelt wird, Energiekosten zu reduzieren. Die meisten Klima- und/oder Belüftungsanlagen werden nämlich entsprechend starren, meist vordefinierten und/oder vorprogrammierten Zyklen betrieben, welche insbesondere zeitgesteuert sind und welche z. B. eine tatsächliche, kaum planbare Raumbelegung nicht berücksichtigen. Diese Anlagen sind daher auch in Betrieb, wenn kein Bedarf besteht. Mit der Steuerung und/oder Regelung einer Klima- und/oder Belüftungsanlage auf Basis des Ausgangssignals, insbesondere auf Basis der tatsächlich herrschenden CO2-Konzentration, können somit bis zu 80 % der notwendigen Energiekosten für den Betrieb einer Klima- und/oder Belüftungsanlage eingespart werden.

Ein weiterer Vorteil der vorliegenden Erfindung kann darin bestehen, dass, insbesondere aufgrund ihrer einfachen Handhabung und/oder der besonders leicht ablesbaren und zu interpretierenden Darstellung der Luftqualität, ein Benutzer sofort, also in Echtzeit, über ein mögliches Gesundheitsrisiko informiert wird. Somit ist die vorliegende Erfindung für die Benutzung durch nahezu alle Personengruppen, beispielsweise auch durch Kinder und/oder durch Menschen mit Behinderung, geeignet.

Ein weiterer Vorteil kann in der Möglichkeit bestehen, defekte Sensoren, Sensormodulen und/oder Sensoranordnungen in einfacher Weise auszutauschen, was das erfindungsgemäßen Sensorsystems vorteilhafterweise äußerst nachhaltig und reparaturfreundlich macht.

Noch ein weiterer Vorteil kann in bestimmten Ausführungsformen darin bestehen, insbesondere aufgrund der kabellosen Übertragung, dass weniger Wartungs- und/oder Servicekosten entfallen, da häufige Verschleißerscheinungen wie Kabelbruch und dadurch resultierende Übertragungsprobleme wegfallen.

Ein weiterer Vorteil der Erfindung kann darin bestehen, dass das erfindungsgemäße Sensorsystem, insbesondere aufgrund der geringen Größe und/oder des geringen Gewichts seiner Erfassungseinrichtung, leicht überall mitgenommen werden kann, z. B. in eine Hand- oder Laptoptasche, im Briefbeutel, im Reisegepäck. Des Weiteren kann das erfindungsgemäße Sensorsystem mit einem externen Gerät wie beispielsweise einem mobilen Telefon oder einem Rechner, verbunden werden kann und von diesen mit Energie versorgt oder mittels diesen aufgeladen werden kann. Somit kann das erfindungsgemäße Sensorsystem praktisch überall nicht nur mithingenommen, sondern auch in Betrieb genommen und verwendet bzw. betrieben werden oder nachgeladen werden. Das erfindungsgemäße Sensorsystem kann daher in beliebiger Umgebung als persönlicher Gesundheitsbegleiter Anwendung finden. Somit kann jeder individuell, unabhängig von lokalen Maßnahmen, beispielsweise im Büro, in öffentlichen Verkehrsmitteln, in einer Versammlung und so weiter, selbst die Luftqualität seiner Umgebung in Echtzeit überprüfen und/oder überwachen. Somit kann jeder Anwender sein persönliches Risiko, ggf. auch das Risiko seiner Familienangehöriger, Freunde, Kollegen, Mitreisender usw., in Echtzeit einschätzen und durch entsprechende Reaktion(en) verringern.

Ein weiterer Vorteil der Erfindung kann darin bestehen, dass in einigen Ausführungsformen eine oder mehrere Erfassungseinrichtung(en) voneinander räumlich getrennt, vorzugsweise zentral, abgefragt, gesteuert, geregelt und/oder überwacht werden können. Dadurch können mit dem Sensorsystem mehrere Umgebungen, beispielsweise verschiedene Zimmer und/oder Personenumgebungen, gleichzeitig und in Echtzeit überwacht werden.

Ein weiterer Vorteil der Erfindung kann darin bestehen, dass die CO2-Konzentration durch entsprechende Maßnahmen, z. B. Belüften, in einem für die Gesundheit unbedenklichen Bereich gehalten werden kann. Dadurch kann beispielsweise eine Verminderung der Leistungsfähigkeit, z. B. von Mitarbeitern, z. B. wegen verminderter Konzentrationsfähigkeit aufgrund einer zu hohen CO2-Konzentration, vorgebeugt werden, wodurch die Produktivität erhöht beziehungsweise aufrechterhalten werden kann.

Ein weiterer Vorteil der Erfindung kann in einigen Ausführungsformen darin bestehen, dass die Messung einer Umweltgröße mittels zwei oder mehr Sensoren es vorteilhaft ermöglicht, offensichtliche Messfehler auszusortieren und/oder eine Messgenauigkeit zu erhöhen und/oder Aussagen über die Funktionalität der Sensoren zu treffen, beispielsweise die Notwendigkeit einen Sensor auszutauschen. Beispielsweise kann die Messung einer Umweltgröße mittels zwei oder mehr Sensoren vorteilhaft die Feststellung einer Abweichung eines Sensors gegenüber einem anderen Sensor, das als Normal bezeichnet wird, ermöglichen. Dadurch kann sichergestellt werden, dass die Überwachung der Umweltgröße zuverlässig ist.

In manchen Ausführungsformen kann die Ermittlung der gleichen Umweltgröße auf verschiedene Messprinzipien erfolgen oder basieren. Dadurch kann sichergestellt werden, dass die gemessenen Werte unabhängig vom Messprinzip im Wesentlichen kongruent sind. Andernfalls kann eine Fehlermeldung herausgegeben werden. Dadurch kann die Zuverlässigkeit des Sensorsystems weiter erhöht werden.

Die Erfassungseinrichtungen können optional zusätzlich miteinander in Datenverbindung, vorzugsweise in drahtloser Verbindung, stehen oder hierfür vorbereitet sein. Vorteilhafterweise kann somit ein vorzugsweise drahtloser Erfassungsnetzwerk gebildet werden, wobei die verteilten Erfassungseinrichtungen drahtlos über kurze Distanzen beispielsweise mittels eines leistungsschwachen Funkmoduls kommunizieren können. Dadurch kann es möglich sein, beispielsweise auch da wo kein WLAN zur Verfügung steht, ein Sensorsystem mit mehreren Erfassungseinrichtungen zu verwenden.

Mit der vorliegenden Erfindung kann vorteilhaft der Status einer Luftqualität ermitteln und/oder signalisieren werden. Dies kann vorteilhaft dazu verwendet werden, anwesende Personen vor schlechter Luftqualität zu warnen, die Verbesserung derselben zu veranlassen und damit gesundheitsschädliche Auswirkungen von schlechter Luftqualität auf die betreffenden Personen zu reduzieren oder zu eliminieren. Dadurch können durch CO2 verursachte höhere Krankheitsquoten, insbesondere von Mitarbeitern, gemindert werden.

Die vorliegende Erfindung wird im Folgenden anhand der beigefügten Zeichnungen, in welcher identische Bezugszeichen gleiche oder ähnliche Bauteile bezeichnen, exemplarisch erläutert. Pfeile mit doppelter Spitze symbolisieren eine drahtlose und/oder kabelgebundene Verbindung, insbesondere eine Datenverbindung. In den zum Teil stark vereinfachten Figuren gilt:
- **Fig. 1**: zeigt in schematischer Darstellung ein Sensorsystem in einer ersten Ausführungsform;
- **Fig. 2**: zeigt die Darstellung einer möglichen Ausführungsform einer Erfassungsvorrichtung eines erfindungsgemäßen Sensorsystems 1;
- **Fig. 3**: zeigt in vereinfachter Darstellung ein Sensorsystem in einer dritten Ausführungsform; und
- **Fig. 4**: zeigt in schematischer Darstellung den Ablauf eines erfindungsgemäßen Verfahrens in einer ersten Ausführungsform.

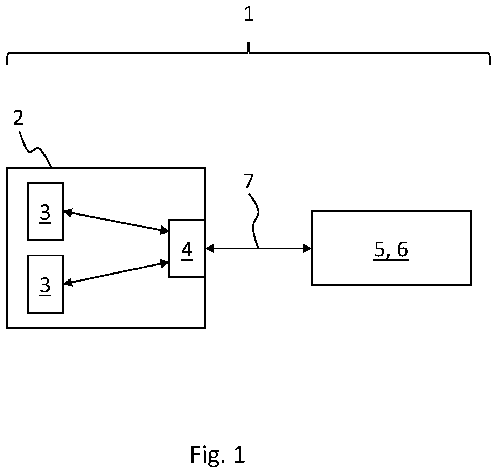

**Fig. 1** zeigt in schematischer Darstellung ein Sensorsystem 1 in einer ersten Ausführungsform.

Das Sensorsystem 1 umfasst eine Erfassungseinrichtung 2, die wenigstens einen Sensor 3 umfasst, der wenigstens zum Ermitteln einer CO2-Konzentration ausgebildet ist. Die Erfassung der CO2-Konzentration erfolgt beispielhaft nach einem Infrarotabsorption-Messprinzip. Bei dieser Messmethode wird insbesondere von der Tatsache Gebrauch gemacht, dass CO2-Moleküle Infrarotstrahlung im Bereich einer Wellenlänge von 4,3 µm absorbieren, während andere Gasmoleküle diesen Spektralbereich elektromagnetischer Strahlung kaum absorbieren. Die Messung der Strahlungsintensität im Bereich einer Wellenlänge von 4,3 µm ermöglicht daher einen Rückschluss auf die CO2-Konzentration.

Optional weist die Erfassungseinrichtung 2 einen oder mehrere weitere Sensoren 3 auf. Beispielsweise weist die Erfassungseinrichtung 2 weiterhin einen nicht dargestellten Luftfeuchtigkeitssensor und/oder einen Temperatursensor auf.

Die Erfassungseinrichtung 2 weist zudem eine Kommunikationsschnittstelle 4 auf, welche beispielsweise mit einer nicht dargestellten Ausgabeeinrichtung 5 der Erfassungseinrichtung und einer Benutzerschnittstelle 6, vorzugsweise drahtlos, verbunden ist oder werden kann. Die Verbindung kann optional mittels einer Leitung, beispielsweise mittels eines Datenkabels erfolgen.

Die Kommunikationsschnittstelle 4 kann alternativ oder ergänzend optional mit einer Software und/oder einem GUI, vorzugsweise drahtlos, in Datenverbindung, insbesondere in Datenaustausch, sein oder stehen. Die Verbindung kann optional drahtlos und/oder mittels einer Leitung, beispielsweise mittels eines Datenkabels erfolgen. Die Software kann beispielsweise eine App oder ein Programm sein, welche oder welches vorzugsweise auf einer externen Vorrichtung oder Einrichtung, beispielsweise einem Smartphone, Rechner und/oder Laptop, ausgeführt und/oder ausführbar und/oder bedienbar ist. Die Software und/oder das GUI kann Teil des Sensorsystems 1 sein. Die Software kann insbesondere die Benutzerschnittstelle 6 sein und vorzugsweise eine Benutzeroberfläche und/oder Anzeigeoberfläche aufweisen, erzeugen und/oder zur Verfügung stellen.

Die Kommunikationsschnittstelle 4 ist zudem verbunden oder wenigstens in Datenverbindung mit dem Sensor 3 oder mit den Sensoren 3. Die Datenverbindung kann jeweils kabelgebunden oder drahtlos erfolgen.

Die Benutzerschnittstelle 6 kann jeweils optional Teil der Erfassungseinrichtung 2, insbesondere des Gehäuses 8 der Erfassungseinrichtung 2 sein. Alternativ kann die Benutzerschnittstelle 6 von der Erfassungseinrichtung 2 unabhängige und/oder räumlich getrennte Einrichtung sein. Die Benutzerschnittstelle kann aus verschiedenen Einrichtungen aus der Gruppe von Ausgabe-, Alarm-, Anzeige-, Betätigungs- und/oder Eingabeeinrichtungen und/oder -vorrichtungen bestehen.

Ausgabe-, Alarm-, Anzeige-, Betätigungs- und/oder Eingabeeinrichtungen können jeweils optional externe Vorrichtungen sein, welche nicht Teil des erfindungsgemäßen Sensorsystems 1 sind, oder solche bilden.

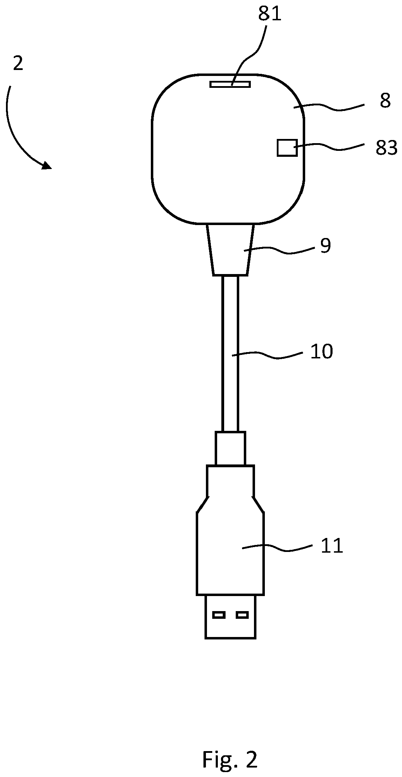

Die Erfassungseinrichtung 2 weist optional eine erste Statusanzeige 81 (siehe Fig. 2), welche einen Betriebsstatus der Erfassungseinrichtung 2 wiedergeben kann. Beispielsweise kann eine solche erste Statusanzeige 81 einen Betriebs-, Sende- und/oder Ladestatus der Erfassungseinrichtung 2 wiedergeben (z. B. Power ON / Power OFF / waiting / sending / low battery usw).

Die Erfassungseinrichtung 2 ist ausgebildet, konfiguriert und/oder programmiert ein Ausgangssignal 7 auf Basis der mit dem Sensor 3 gewonnenen Messdaten zu erzeugen und mittels der Kommunikationsschnittstelle 4 wenigstens an eine Benutzerschnittstelle 6 zu übermitteln. Die Erfassungseinrichtung 2 ist optional ausgebildet, konfiguriert und/oder programmiert, um Daten einer Benutzerschnittstelle 6 zu erhalten, empfangen und/oder zu verarbeiten.

Das Ausgangssignal 7 kann wenigstens die mit dem Sensor 3 ermittelten Daten umfassen und/oder wenigstens ein Signal und/oder eine Aussage über die mit dem Sensor 3 ermittelten Daten umfassen. Beispielsweise kann das Ausgangssignal 7 die Information umfassen, dass die mit dem Sensor 3 ermittelten Daten einen bestimmten Wert oder Wertebereich unter- oder überschreiten. Das Ausgangssignal 7 kann optional mittels einer drahtlosen Verbindung oder mittels einer Kabelverbindung an die Benutzerschnittstelle 6, beispielsweise eine Anzeigeeinrichtung, übermittelt werden.

Eine Anzeigeeinrichtung kann insbesondere ausgebildet, konfiguriert und/oder programmiert sein, das Ausgangssignal 7 der Erfassungseinrichtung 2 anzuzeigen, wiederzugeben bzw. darzustellen.

Die Erfassungseinrichtung 2 kann optional eine Ausgabeeinrichtung (nicht dargestellt) umfassen, welche vorzugsweise zwischen dem Sensor oder den Sensoren 3 und der Kommunikationsschnittstelle 4 angeordnet und/oder zwischengeschaltet ist, und welche wenigstens ein Ausgangssignal 7 auf Basis der mit dem Sensor 3 ermittelten Sensordaten erzeugen und/oder bereitstellen kann.

Beispielsweise kann eine Benutzereinrichtung 6 eine LED-Anzeige und/oder ein Display, beispielsweise ein Touchscreen, sein und/oder umfassen. Insbesondere kann eine Benutzereinrichtung 6 ein Unter- oder Überschreiten eines bestimmten Werts oder Wertebereichs farblich unterschiedlich und/oder zahlenmäßig wiedergeben. Beispielsweise kann die Anzeige oder die Wiedergabe als "Ampel" ausgebildet sein und grün leuchten, wenn eine gemessene CO2-Konzentration sich in einem Wertebereich unterhalb von 800 ppm (parts per million, 10⁻⁶) befindet, orange leuchten, wenn eine gemessene CO2-Konzentration sich in einem Wertebereich zwischen 800 ppm und 1000 ppm befindet und rot leuchten, wenn eine gemessene CO2-Konzentration sich in einem Wertebereich über 1000 ppm, insbesondere über 1200 ppm, befindet. Alternativ oder ergänzend können optional die Echtzeitwerte, oder manche Echtzeitwerte der gemessenen Umweltgrößen und/oder weitere darauf basierende Signale und/oder Hinweise, dargestellt und/oder angezeigt werden.

Eine Benutzereinrichtung 6 kann alternativ oder ergänzend und optional eine akustische Ausgabe, beispielsweise ein Piepsen, und/oder eine haptische Ausgabe, beispielsweise eine Vibration und/oder ein Vibrationsmuster, herausgeben, wenn beispielsweise bestimmte Grenzwerte über- und/oder unterschritten werden.

Die Erfassungseinrichtung 2 kann wenigstens eine nicht weiter dargestellte Energie-, Strom- und/oder Spannungsquelle umfassen. Alternativ oder ergänzend kann die Erfassungseinrichtung 2 mit wenigstens einer Energie-, Strom- und/oder Spannungsquelle verbindbar sein.

Die Erfassungseinrichtung 2 kann wenigstens einen nicht weiter dargestellten Datenspeicher und/oder wenigstens eine nicht weiter dargestellte Steuer- und/oder Regeleinrichtung umfassen. Alternativ oder ergänzend kann die Erfassungseinrichtung 2 mit wenigstens einem nicht weiter dargestellten Datenspeicher und/oder mit wenigstens einer nicht weiter dargestellten Steuer- und/oder Regeleinrichtung verbindbar sein.

**Fig. 2** zeigt eine mögliche Ausführung einer Erfassungseinrichtung 2 des erfindungsgemäßen Sensorsystems 1.

Die Erfassungseinrichtung 2 weist ein Gehäuse 8 auf.

Das Gehäuse 8 weist beispielhaft zwei Statusanzeigen auf. Eine erste Statusanzeige 81 ist exemplarisch oben am Gehäuse 8 der Erfassungseinrichtung 2 angeordnet. Die erste Statusanzeige 81 kann beispielsweise eine optische Anzeige sein und/oder einen Betriebszustand der Erfassungseinrichtung 2 wiedergeben. Beispielsweise leuchtet die Anzeige farbig, beispielsweise grün, wenn diese in Betrieb und/oder mit einer nicht weiter dargestellten Energie-, Strom- und/oder Spannungsquelle verbunden ist. Eine zweite Statusanzeige 83, die in Fig. 2 rechts am Gehäuse 8 angeordnet ist, kann beispielweise dann leuchten, wenn die Erfassungseinrichtung 2 mit einer externen Vorrichtung, beispielsweise drahtlos, verbunden ist.

Das Gehäuse 8 der Erfassungsvorrichtung 2 kann beliebige Dimensionen und/oder Formen haben, vorzugsweise ist das Gehäuse im Wesentlichen rechteckig und/oder oval und es kann beispielsweise die Abmessungen 36 mm * 36 mm * 16mm aufweisen.

Das Gehäuse kann zudem wenigstens einen Anschluss 9 für eine Verbindungsleitung 10 aufweisen. In bestimmten Ausführungsformen kann die Verbindungsleitung 10 vom Gehäuse 8 abnehmbar sein. Alternativ kann die Verbindungsleitung 10 fester Bestandteil der Erfassungseinrichtung 2 sein, insbesondere nicht trennbar vom Gehäuse 8 ausgebildet sein.

Die Verbindungsleitung 10 kann eine Datenkommunikations- und/oder eine Versorgungsleitung, insbesondere eine Energie-, Strom- und/oder Spannungsversorgungsleitung sein.

Vorzugsweise ist die Verbindungsleitung 10 mit einer Kommunikationsschnittstelle 4 (in Fig. 2 nicht gezeigt) der Erfassungseinrichtung 2 verbunden.

Die Verbindungsleitung 10 kann wenigstens einen Anschluss 11 zum Verbinden mit wenigstens einer externen Einrichtung und/oder Vorrichtung und/oder mit wenigstens einer Benutzerschnittstelle 6 aufweisen und/oder wenigstens einen Anschluss 11 zum Verbinden mit einer externen Energie-, Strom- und/oder Spannungsversorgungsleitung aufweisen. Im Beispiel der Fig. 2 ist ein USB-Stecker als Anschluss 11 angedeutet. Der Anschluss 11 kann aber optional jeden bekannten Anschluss sein oder entsprechen, insbesondere einen üblichen Standardanschluss, z. B. für einen Rechner oder ein mobiles Telefongerät.

Die Erfassungseinrichtung 2 kann optional mehrere abnehmbare Verbindungsleitungen 10 mit unterschiedlichen Anschlüssen 11 zum Verbinden mit einer externen Einrichtung und/oder Vorrichtung aufweisen.

Beispielweise kann eine Verbindungsleitung 10 als Anschluss 11 einen USB-, Micro-USB- oder USB-C-Stecker, einen Anschluss für ein Apple-Gerät, beispielsweise Apple Lightning, und/oder einen sonstigen bekannten Anschluss, vorzugsweise einen Standard- und/oder proprietären Anschluss aufweisen. Optional kann alternativ die Verbindungsleitung 10 mit entsprechenden Adaptern verbindbar sein und/oder geliefert werden, welche dann Teil des Sensorsystems 1 sein können aber nicht müssen.

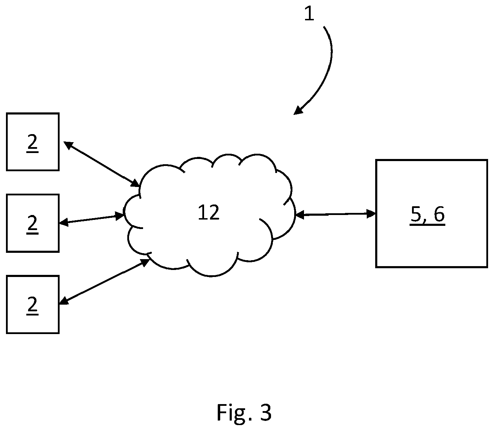

**Fig. 3** zeigt in vereinfachter Darstellung ein Sensorsystem 1 in einer weiteren Ausführungsform.

Das Sensorsystem 1 weist dabei mehrere Erfassungseinrichtungen 2 auf. Die Erfassungseinrichtungen 2 können identisch sein oder verschieden ausgebildet sein, insbesondere können sie beispielsweise verschiedene Sensoren und/oder Sensoren-Kombinationen, insbesondere Sensoren verschiedener Wirk- und/oder Messweisen und/oder in verschiedenen Anordnungen, aufweisen.

Die Erfassungseinrichtungen 2 können optional zusätzlich miteinander in Datenverbindung, vorzugsweise in drahtloser Verbindung, stehen oder hierfür vorbereitet sein. Vorteilhafterweise kann somit ein vorzugsweise drahtloser Erfassungsnetzwerk gebildet werden, wobei die verteilten Erfassungseinrichtungen 2 drahtlos über kurze Distanzen beispielsweise mittels eines leistungsschwachen Funkmoduls kommunizieren können.

Die Erfassungseinrichtungen 2 weisen jeweils wenigstens eine Kommunikationsschnittstelle 4 auf und/oder sind ausgebildet und/oder konfiguriert, jeweils ein Ausgangssignal 7 zu erzeugen.

Beispielhaft sind die Erfassungseinrichtungen 2 mittels eines Netz- und/oder Funkwerkes 12 mit wenigstens einer Benutzerschnittstelle 6, insbesondere mit wenigstens einer Ausgabe-, Alarm-, Anzeige-, Betätigungs- und/oder Eingabeeinrichtung und/oder einer externen Vorrichtung verbunden und/oder in Datenverbindung.

Somit können vorteilhaft beispielsweise verschiedene Räume oder Raumteile überwacht werden und/oder die Erfassungseinrichtungen 2 zentral gesteuert und/oder geregelt werden.

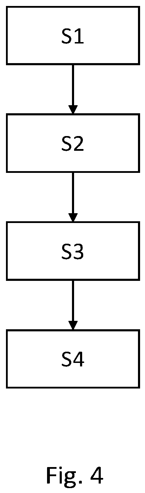

**Fig. 4** zeigt schematisch ein Verfahren in einer ersten Ausführungsform.

In einem ersten Schritt S1 werden Grenzwerte und/oder Wertebereiche für wenigstens eine Umweltgröße und/oder Umweltdaten bereitgestellt, ermittelt.

In einem weiteren Schritt S2 wird wenigstens ein Wert einer Umweltgröße mittels einer Erfassungseinrichtung 2 eines erfindungsgemäßen Sensorsystems ermittelt.

In einem weiteren Schritt S3 wird der wenigstens eine Wert der Umweltgröße mit den vorher ermittelten, eingegebenen, und/oder bereitgestellten Grenzwerte und/oder Wertebereiche verglichen.

In einem weiteren Schritt S4 wird ein Ausgangssignal auf Basis des ermittelten Werts der Umweltgröße und/oder auf Basis des Vergleichs diesen Wert mit den vorher ermittelten, eingegebenen und/oder bereitgestellten Grenzwerte und/oder Wertebereiche erzeugt.

Ein erfindungsgemäßes Verfahren kann weitere früher beschriebene Schritte in beliebiger Reihenfolge umfassen.

### Bezugszeichenliste

- 1: Sensorsystem
- 2: Erfassungseinrichtung
- 3: Sensor(en), Sensormodul(e) oder 3 Sensoranordnung(en)
- 4: Kommunikationsschnittstelle
- 5: Anzeigeeinrichtung
- 6: Benutzerschnittstelle
- 7: Ausgangssignal
- 8: Gehäuse
- 81: erste Statusanzeige
- 83: zweite Statusanzeige
- 9: Anschluss für eine Verbindungsleitung
- 10: Verbindungsleitung
- 11: Anschluss zum Verbinden mit einer externen Einrichtung und/oder Vorrichtung
- 12: Cloud; Netzwerk; Funknetz

- S1 bis S4: Verfahrensschritte

## Patentansprüche

1. Sensorsystem (1) zum Überwachen wenigstens einer Umweltgröße mit wenigstens einer
Erfassungseinrichtung (2), umfassend wenigstens einen Sensor (3), der wenigstens zum Ermitteln wenigstens einer Umweltgröße, insbesondere wenigstens einer CO2-Konzentration, ausgebildet ist,
wobei die Erfassungseinrichtung (2) wenigstens eine Kommunikationsschnittstelle (4) aufweist,
und/oder
wobei das Sensorsystem (14) ausgebildet und/oder konfiguriert ist, ein Ausgangssignal (7) zu erzeugen, welches wenigstens einen ermittelten Wert der wenigstens einen Umweltgröße berücksichtigt.

2. Sensorsystem (1) nach Anspruch 1, wobei das Sensorsystem (1) und/oder die Erfassungseinrichtung (2) wenigstens eine Verbindungsleitung (10) aufweist.

3. Sensorsystem (1) nach einem der vorhergehenden Ansprüche, wobei die Kommunikationsschnittstelle (4) wenigstens eine drahtlose Kommunikationsverbindung und/oder wenigstens eine Kommunikationsleitung ist oder umfasst.

4. Sensorsystem (1) nach einem der vorhergehenden Ansprüche, wobei die Kommunikationsschnittstelle (4) wenigstens eine Sende- und/oder Empfangseinrichtung aufweist ist oder umfasst, und/oder mit wenigstens eine Sende- und/oder Empfangseinrichtung in Datenverbindung steht.

5. Sensorsystem (1) nach einem der vorhergehenden Ansprüche, wobei das Sensorsystem (1) und/oder die Erfassungseinrichtung (2) wenigstens einen Datenspeicher umfassen.

6. Sensorsystem (1) nach einem der vorhergehenden Ansprüche, wobei die Erfassungseinrichtung (2) wenigstens ein Gehäuse (8) umfasst, das wenigstens einen Anschluss (11) für eine Verbindungsleitung (10) aufweist.

7. Sensorsystem (1) nach einem der vorhergehenden Ansprüche, wobei das Sensorsystem (1) und/oder die Erfassungseinrichtung (2) wenigstens eine Benutzerschnittstelle (6) umfasst, und/oder mit einer solchen in Daten- und/oder Kommunikationsverbindung verbunden ist.

8. Sensorsystem (1) nach einem der vorhergehenden Ansprüche, wobei das Sensorsystem (1) und/oder die Erfassungseinrichtung (2) wenigstens eine Einrichtung (5) zum Ausgeben wenigstens eines Ausgangssignals umfasst, welche ausgebildet, konfiguriert und/oder programmiert ist, zumindest ein Ausgangsignal wenigstens auf Basis der mit der Erfassungseinrichtung ermittelten Daten zu erzeugen.

9. Sensorsystem (1) nach einem der vorhergehenden Ansprüche, wobei die Erfassungseinrichtung (2) wenigstens einen CO-Sensor, wenigstens einen CO2-Sensor, wenigstens einen Temperatursensor, wenigstens einen Luftdrucksensor, und/oder wenigstens einen Feuchtigkeitssensor ist oder umfasst.

10. Verfahren zum Überwachen wenigstens einer Umweltgröße und/oder zum Ausgeben eines Ausgangssignals umfassend wenigstens folgenden Schritt:
- Ermitteln wenigstens eines Wertes einer Umweltgröße; und/oder
- Ausgeben eines Signals oder Hinweises, welcher wenigstens einen ermittelten Wert der wenigstens einen Umweltgröße berücksichtigt.

11. Steuer- und/oder Regeleinrichtung geeignet und vorgesehen und/oder ausgelegt und/oder konfiguriert zur Durchführung des Verfahrens nach Anspruch 10.

12. Digitales Speichermedium, insbesondere in Form einer Diskette, CD oder DVD oder EPROM, mit elektrisch auslesbaren Steuersignalen, konfiguriert, um derart mit einem programmierbaren Computersystem zusammenzuwirken, dass die maschinellen Schritte eines erfindungsgemäßen Verfahrens nach Anspruch 10 veranlasst werden.

13. Computerprogramm-Produkt mit einem auf einem maschinenlesbaren Träger gespeicherten Programmcode zur Veranlassung der maschinellen Schritte des erfindungsgemäßen Verfahrens gemäß Anspruch 10, wenn das Computerprogramm-Produkt auf einem Rechner abläuft.

14. Computerprogramm mit einem Programmcode zur Veranlassung der maschinellen Schritte eines erfindungsgemäßen Verfahrens gemäß Anspruch 10, wenn das Computerprogramm auf einem Computer abläuft.
